Europäisches Patentamt
European Patent Office
Office européen des brevets

(19)

(11) Publication number: **0 385 348 A1**

(12) # EUROPEAN PATENT APPLICATION

(21) Application number: 90103701.0

(22) Date of filing: 26.02.90

(51) Int. Cl.[5]: C07D 493/04, A61K 31/44, //(C07D493/04,307:00,307:00)

(30) Priority: 28.02.89 DE 3906267

(43) Date of publication of application: 05.09.90 Bulletin 90/36

(84) Designated Contracting States: AT BE CH DE DK ES FR GB GR IT LI LU NL SE


(71) Applicant: Heinrich Mack Nachf.
Postfach 2064
D-7918 Illertissen(DE)

(72) Inventor: Stoss, Peter, Dr.
Mozartstrasse 15
D-7918 Illertissen(DE)
Inventor: Hemmer, Reinhard, Dr.
Rosenstrasse 2a
D-7918 Illertissen-Au(DE)
Inventor: Merrath, Peter
Augsburger Strasse 9
D-8946 Memmingerberg(DE)

(74) Representative: Lederer, Franz, Dr. et al
Lederer, Keller & Riederer, Patentanwälte,
Lucile-Grahn-Strasse 22
D-8000 München 80(DE)


(54) **Pyridylcarboxyl-isosorbide derivative, manufacturing process and uses.**


(57) The present invention concerns sornidipine monohydrate of the empirical formula $C_{22}H_{26}N_2O_{10}$ and the structural formula

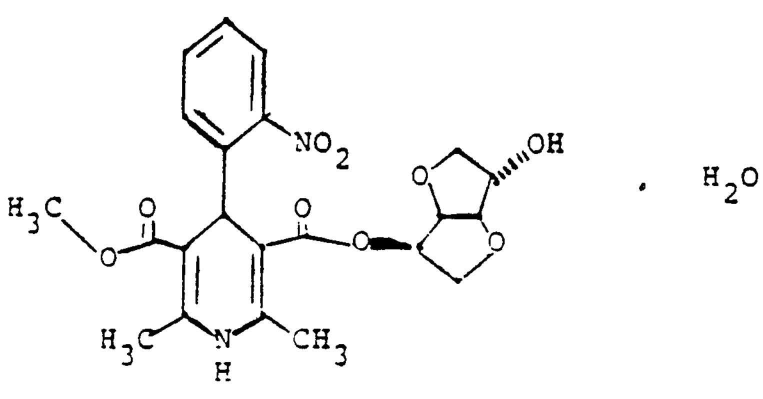

a process for its manufacture, its use in treating hypertension, pulmonary hypertension and angina pectoris and pharmaceutical compositions comprising sornidipine monohydrate.


EP 0 385 348 A1

# PYRIDYLCARBOXYL-ISOSORBIDE DERIVATIVE, MANUFACTURING PROCESS AND USES

## Field of the Invention

The present invention concerns a new pyridylcarboxylisosorbide derivative, namely sornidipine monohydrate, its manufacturing process and its use in the treatment of hypertension, pulmonary hypertension and angina pectoris.

## Background of the Invention

In the European patent 114 270, new acyl derivatives of 1,4:3,6-dianhydro-hexitols are described, which are suitable for pharmaceutical use. All the compounds named as working examples in this document exist as anhydrous substances. Among those there is one which in the meantime, under the international non-proprietary name sornidipine, has been the subject of intensive experimental evaluation as a cardiovascular drug. Sornidipine is S(+)-5-[1,4-dihydro-2,6-dimethyl-3-methoxycarbonyl-4-(2-nitrophenyl)-5-pyridylcarbonyl]-isosorbide.

Surprisingly, it has now been discovered that sornidipine is capable of forming a stable monohydrate. This finding can be regarded as completely unexpected. From the literature, no other examples are known so far which describe compounds of similar chemical structure in the form of defined hydrates. This can be confirmed by the following listed marketed or developmental compounds of this structure type, which all appear in the literature as anhydrous forms only, at the most as salts but never as hydrates: Amlodipine, benidipine, darodipine, elnadipine, felodipine, flordipine, isradipine, lacidipine, manidipine, mesudipine, nicardipine, nifedipine, niguldipine, niludipine, nilvadipine, nimodipine, nisoldipine, nitrendipine, oxodipine, and ryodipine.

## Summary of the Invention

The subject of the present invention is sornidipine monohydrate with the following structural formula:

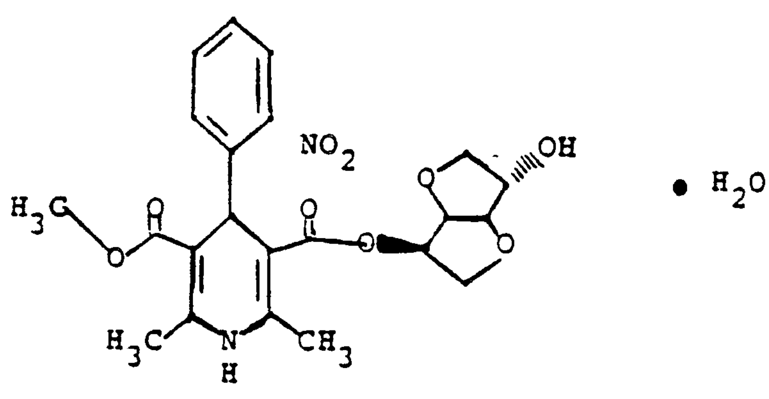

and the empirical formula $C_{22}H_{24}N_2O_9 \cdot H_2O$ or $C_{22}H_{26}N_2O_{10}$ and the molecular weight of 478.46.

The theoretical water content of the monohydrate is 3.76%.

## Detailed Description of the Invention

The manufacture of sornidipine hydrate can be carried out, for example, by recrystallizing the anhydrous substance from solvent mixtures which contain at least that quantity of water necessary for the

formation of the hydrate. The amount of water can, however, be greater. As organic solvents usable in a mixture with water all those are possible which, on the one hand, possess good soluting properties for sornidipine and which, on the other hand, are miscible with at least that quantity of water required to achieve the formation of a monohydrate.

The following solvents are examples for this: lower alcohols, such a methanol, ethanol, propanol and isopropanol, lower ketones such as acetone and methylethylketone, ethers, such as ethyleneglycol-monomethylether, -monoethylether, -dimethylether, diethyleneglycol-dimethylether, tetrahydrofurane, dioxane and other aprotic solvents such as acetonitrile, N,N-dimethylformamide, dimethylsulfoxide and sulfolane.

Solvent mixtures of preference are those consisting of methanol/water, ethanol/water, propanol/water, isopropanol/water and acetone/water.

The water content in the solvent mixture may vary from the minimum amount necessary for the monohydrate formation of a given amount of sornidipine to the limit of miscibility of the organic solvent with water. In practice, mixture ratios of organic solvent:water between 10:1 and 1:10 would mainly be used, whereby mixture ratios of 5:1 to 1:5 are preferable.

The crystallization is carried out in such a way that the anhydrous sornidipine is dissolved in the required amount of solvent/water mixture at a temperature between room temperature and the boiling point of the particular solvent/water mixture. Crystallization of sornidipine monohydrate is induced by reduction of temperature. In one preferred method, sornidipine is completely dissolved in the required amount of solvent/water mixture at the boiling point. The solvent is then filtered, if necessary, and sornidipine monohydrate is subsequently crystallized by cooling to room temperature or ice bath temperature, if necessary while stirring.

The product obtained in this way can then be isolated by filtration, centrifugation or by any other suitable separation technique. Any adherent solvent or excess of water is subsequently removed from the sornidipine monohydrate by drying.

The drying can take place either under normal pressure or in vacuo and between 0 and 50 °C, but preferably at room temperature, In the drying process, adherent excess water and organic solvent are removed but the water in the crystal lattice, equivalent to the monohydrate, remains in the molecule.

Alternatively, the adherent excess water can also be successfully removed by washing or stirring the separated crystalline product with water-miscible organic solvents. For this purpose, solvents as described above for the recrystallization in a mixture with water can be taken into consideration. The adherent organic solvent is subsequently removed by drying, as above.

Sornidipine monohydrate forms yellow crystals containing one mol of water in a defined from in the crystal lattice as water of crystallization. This has been confirmed, among other methods, by X-ray structure analysis and $^1$H-NMR- and IR-spectra. In contrast to excess, absorptively bound water, the water of crystallization adheres very firmly to the molecule. It can be removed by drying only with great difficulty and not without changing the structure of the crystals.

Naturally, the pharmacological properties of sornidipine monohydrate do not differ from those of the anhydrous form.

Surprisingly, however, in comparison to the anhydrous form described previously, sornidipine monohydrate possesses advantageous properties which facilitate the processing of the substance into various drug forms. It exists therefore, in contrast to the anhydrous form, as a stable, easily flowing crystalline product of very uniform particle size, without a tendency towards electrostatic charging and with increased light stability compared to the anhydrous form.

As is commonly known, dihydropyridines of this structure type, as far as they have a nitro group in 2-or 3- position on the aromatic ring, are subject to light induced degradation. This applies also to sornidipine. However, this degradation is distinctly decelerated with sornidipine monohydrate in comparison with the anhydrous form. This decreased tendency towards photochemical degradation and the resultant simplified handling of this material, also represents an important improvement.

A further advantage of the compound of the invention is that for its crystallization, water-containing solvents are used, which are less dangerous to handle than pure organic solvents. In contrast, the anhydrous form of sornidipine has to be crystallized from organic solvents which are, in most cases, either combustible or easily flammable and therefore represent a greater safety risk.

Furthermore, the anhydrous form of sornidipine has a tendency to tenaciously retain varying quantities of organic solvent which can only be removed, and sometimes even then only unsatisfactorily, by intensive, usually laborious drying in vacuo. Adherent solvents can, however, complicate or even prevent the use of the material for pharmaceutical manufacture. Sornidipine monohydrate does not have this disadvantage. Here, the water-containing organic solvents used for the manufacture or recrystallization can be easily and

completely removed.

Sornidipine monohydrate is therefore excellently qualified for its use in various pharmaceutical application forms. In particular, it can be processed not only to tablets, capsules, film-coated tablets, powders and suppositories but also to syrups or other liquid dosage forms and transdermal systems, if necessary with the addition of the usual excipients.

In addition to sornidipine monohydrate and its manufacture, the present invention concerns therefore also its use in the manufacture of drugs, its use as a drug and drugs containing sornidipine monohydrate. The processing or the substance of the invention into the respective drug forms is carried out according to methods familiar to those skilled in the art and which therefore require no further description.

The dose of active substance can vary, depending on the symptoms, the type and severity of the illness, and the age and weight of the patient and the decision of the physician. In general, however, doses between 1 and 400 mg would be appropriate for an adult human, whereby these quantities could be administered once to several times daily. Drug forms containing this active component in amounts from 2 to 100 mg. especially 5-50 mg are of particular interest.

As already in disclosed EP 114 270, the new compounds described there possess valuable pharmacological properties. They can, for example, be used as antihypertensive agents, as peripheral and central vasodilators and as coronary therapeutics in the treatment of mammals, in particular of humans. These properties apply especially to sornidipine monohydrate. In particular, it can be used for the treatment of hypertension, pulmonary hypertension and angina pectoris. As used herein, the word includes both therapeutic and prophylactic treatment.

The following examples serve to illustrate the invention.

## Example 1

With the exclusion of light, or under sodium light, 500 g of anhydrous sornidipine are suspended in 6.2 1 of a mixture of 5 parts volume of ethanol and 1 part volume of demineralized water and heated to the boiling point in an atmosphere of protective gas (nitrogen or argon). The yellow-orange, clear solution can be filtered if necessary.

Subsequently, the solution is allowed to cool to room temperature, whilst stirring, then to ice bath temperature and the mixture is left for about 2 hours at this temperature. The precipitated crystals can then be filtered off via a glass frit (G3), without protection from light, and the crystals subsequently washed twice with 400 ml each of the solvent mixture previously cooled to 0 °C. The crystals can then be dried in vacuo. Fine, well formed crystals are obtained.

Yield: 446 g
Melting point: 233 °C
Sornidipine monohydrate: $C_{22}H_{26}N_2O_{10}$
Molecular weight: 478.46

| | calculated | found |
|---|---|---|
| C | 55.23 | 55.28 % |
| H | 5.48 | 5.48 % |
| N | 5.85 | 5.94 % |

Water content:
calculated: 3.76%
found: 3.74% (according to KF-method)

## Example 2

22 g of anhydrous sornidipine are dissolved, as described in example 1, in 750 ml of a mixture consisting of one part volume of ethanol and five parts volume of water by warming the mixture to 90 °C. The clear, yellow solution is quickly cooled to lower temperatures by means of an ice bath and stirred for about 4 hours at ice bath temperature, whereby the monohydrate precipitates as a yellow, crystalline mass. After separation of the solvent, by means of filtration, and drying of the crystals (see example 1) 21 g of

monohydrate are obtained.

Example 3

45 g of anhydrous sornidipine are heated to 60 °C in a mixture of four parts volume of acetone and one part volume of water, producing a clear solution. This is slowly cooled to 0 °C while stirring. The precipitating crystalline mass is processed as described in examples 1 and 2.

Yield 42 g

The properties of the compounds obtained in examples 2 and 3 are identical to those described in example 1.

**Claims**

1. Sornidipine monohydrate with the empirical formula $C_{22}H_{24}N_2O_9$ $H_2O$ and the structural formula

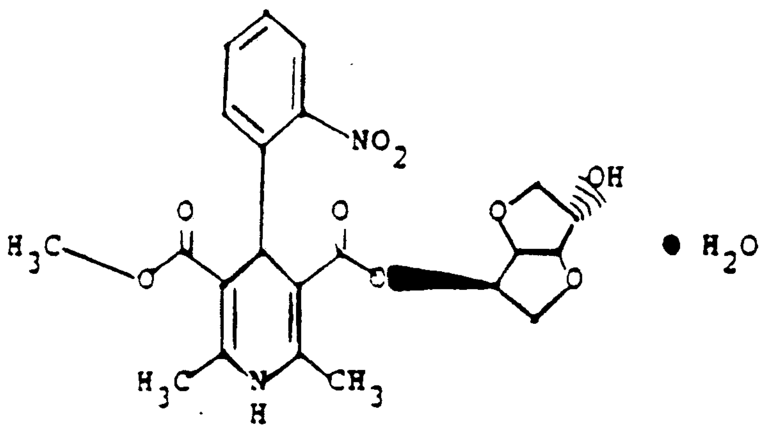

2. Sornidipin monohydrate according to claim 1 for use in the prophylaxis and treatment of diseases.

3. The use of the compound of claim 1 in the manufacture of pharmaceutical compositions for the prophylaxis and treatment of cardiovascular disease.

4. A pharmaceutical composition comprising a therapeutically-effective amount of the compound of claim 1 and, optionally, a pharmaceutically acceptable excipient or carrier agent.

5. A process for the manufacture of sornidipine monohydrate with the empirical formula $C_{22}H_{24}N_2O_9 \cdot H_2O$ and the structural formula

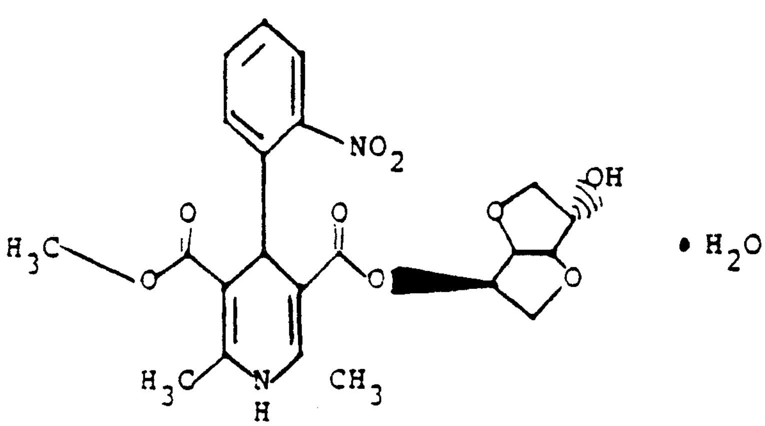

comprising the recrystallization of anhydrous sornidipine from solvent mixtures consisting of a water-miscible organic solvent and water.

6. The process of claim 5, wherein the water-miscible organic solvent is an aprotic solvent.

7. The process of claim 5, wherein the water-miscible organic solvents are selected from the group consisting of lower alcohols, lower ketones, and ethers.

8. The process of claim 5 wherein the water-miscible organic solvents are selected from the group consisting of methanol, ethanol, propanol, isopropanol, acetone, ethyleneglycol-monomethylether, ethyleneglycol-monoethylether, ethyleneglycol-dimethylether, diethyleneglycol-dimethylether, tetrahydrofurane, dioxane, acetonitrile, N,N-dimethylforamide, dimethylsulfoxide and sulfolane.

9. The process of claim 6, wherein the water-miscible organic solvents are selected from the group consisting of methanol, ethanol, propanol, isopropanol, acetone, ethyleneglycol-monomethylether, ethyleneglycol-monoethylether, ethyleneglycol-dimethylether, diethyleneglycol-dimethylether, tetrahydrofurane, dioxane, acetonitrile, N,N-dimethylforamide, dimethylsulfoxide and sulfolane.

10. The process of claim 5, wherein the ratio of organic solvent to water lies in the range of 10:1 to 1:10.

11. The process of claim 10 wherein the ratio of organic solvent and water lies in the range 5:1 to 1:5.

12. The process of claim 6, wherein the ratio of organic solvent to water lies in the range of 10:1 to 1:10.

13. The process of claim 12 wherein the ratio of organic solvent to water lies in the range 5:1 to 1:5.

14. The process of claim 7, wherein the ratio of organic solvents to water lies in the range of 10:1 to 1:10, preferably in the range of 5:1 to 1:5.

15. The process of claim 14 wherein the ratio of organic solvent to water lies in the range 5:1 to 1:5.

European Patent Office

# EUROPEAN SEARCH REPORT

Application Number

EP 90 10 3701

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| A,D | EP-A-0 114 270 (HEINRICH MACK NACHF.)<br>* example 24; claims 1,6,10,11 *<br>--- | 1-4 | C 07 D 493/04<br>A 61 K 31/44 //<br>(C 07 D 493/04<br>C 07 D 307:00 ) |
| A | ACTA CHEMICA SCANDINAVICA<br>vol. B40, 1986, pages 776-778, Oslo, Norway; R. FOSSHEIM: "X-Ray Analysis of Ca2+ Antagonists: 3,5-Bis-(methoxycarbonyl)-2,6-dimethyl-4-(2-aminophenyl)-1,4-dihydropyridine Hydrate"<br>----- | | |

| TECHNICAL FIELDS SEARCHED (Int. Cl.5) |
| --- |
| C 07 D 493/00<br>A 61 K 31/00<br>C 07 D 211/00 |

The present search report has been drawn up for all claims

| Place of search | Date of completion of the search | Examiner |
| --- | --- | --- |
| BERLIN | 05-06-1990 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)